# EUROPÄISCHE PATENTSCHRIFT

(11) **EP 1 212 160 B1**
(45) Veröffentlichungstag und Bekanntmachung des Hinweises auf die Patenterteilung: **03.12.2003**
(21) Anmeldenummer: 99941358.6
(22) Anmeldetag: 14.09.1999
(51) Int. Cl.: B22F 3/11

(54) **GEMISCH AUS ZWEI TEILCHEN-PHASEN ZUR HERSTELLUNG EINES BEI HÖHEREN TEMPERATUREN SINTERFÄHIGEN GRÜNLINGS**
MIXTURE OF TWO PARTICULATE PHASES USED IN THE PRODUCTION OF A GREEN COMPACT THAT CAN BE SINTERED AT HIGHER TEMPERATURES
MELANGE DE DEUX PHASES PARTICULAIRES POUR LA PRODUCTION D'UN COMPACT VERT APTE AU FRITTAGE A DES TEMPERATURES ELEVEES

(43) Veröffentlichungstag der Anmeldung: 12.06.2002
(73) Patentinhaber: Stratec Medical AG, 4436 Oberdorf (CH)
(72) Erfinder: FEICHTINGER, Heinrich, CH-8005 Zürich (CH)
(74) Vertreter: Lusuardi, Werther Giovanni, Dr.
(86) Internationale Anmeldenummer: CH9900434
(87) Internationale Veröffentlichungsnummer: WO01019556

(56) Entgegenhaltungen:
- DE-A- 2 256 716
- DE-C- 19 725 210
- US-A- 3 852 045

## Beschreibung

Der Erfindung betrifft ein Gemisch gemäss dem Oberbegriff des Anspruchs 1, sowie Verwendungen desselben.

Es besteht das Problem, dass offenporige metallische Strukturen, insbesondere wenn sie höhere Porenvolumina und damit zwangsläufig extrem dünnwandige Zellwände enthalten, infolge der hohen spezifischen Oberfläche äusserst sinteraktiv sind. Soll eine solche Struktur, ausgehend von kleineren metallischen Partikeln, zu einem mechanisch ausreichend festen und dichten Formkörper versintert werden, so bedingt dies für dreidimensional ausgedehnte Phasen in der Regel Temperaturen, welche mindestens bei 75 - 80% der Schmelztemperatur liegen. Diese Temperatur muss gewählt werden, da erst in diesem Bereich die Volumsdiffusion, weiche für die Endverdichtung der gesinterten Struktur nötig ist, ausreichend schnell funktioniert. Eine offenporige Metallstruktur mit hohem Porositätsgrad besteht jedoch zu einem wesentlichen Anteil aus inneren Oberflächen und daher ist ihr Sinterverhalten bis zum Schluss weitgehend durch Oberflächendiffusion bestimmt, welche eine wesentlich geringere thermische Aktivierung benötigt. Bei den obengenannten Sintertemperaturen, wie sie normalerweise für die Erzeugung einer dichten metallischen Struktur nötig sind, kommt es daher unausweichlich zu einer Verkleinerung der inneren Oberfläche. Unter der Wirkung der Oberflächenspannung kommt es daher zu einer mehr oder weniger grossen Schwindung des Formkörpers, wodurch einerseits die Dimensionstreue leidet und andererseits das Porenvolumen abnimmt. Dieser Prozess kann verhindert werden, indem man die Sintertemperatur zu tieferen Werten absenkt, welche der thermischen Aktivierung der Oberflächendiffusion entsprechen. Es gelingt dann zwar, die Makrogeometrie des Formkörpers weitgehend beizubehalten, jedoch kommt es lediglich zur Bildung dünner Sinterhälse zwischen benachtbarten Metallpartikeln und der Formkörper weist deshalb nur eine ungenügende Festigkeit auf. Der hier beschriebene Effekt macht es praktisch unmöglich, feinere offenporige Formkörper, deren Zellwände im Bereich von einigen wenigen Mikrometern und deren Porendurchmesser unter 0.5 mm liegen, auf normalem Wege zu sintern: die Oberflächenspannung als treibende Kraft wird dafür sorgen, dass im ursprünglichen Grünkörper vorhandene Poren immer kleiner und die Zellwände immer dicker werden.

In der metallischen und keramischen Technik gibt es zahlreiche Methoden zur Erzeugung offenporiger Formkörper. Die einfachste Methode beruht darauf, ein möglichst lockeres Haufwerk von Partikeln mit einem geeigneten Binder zu einem Formkörper zu pressen und diesen anschliessend zu sintern. Liegt die Dichte dabei unterhalb von 95% des theoretischen Werts, so ist der Körper automatisch offenporig. Solcherart hergestellte Körper, wie sie typischerweise z.B. für selbstschmierende Lager hergestellt werden, bestehen jedoch aus einem Porennetzwerk, welches die negative Abbildung der es umgebenden Metallpartikel ist. Es handelt sich also um enge und scharfkantige Kanäle und da der Zusammenhalt der Partikel durch Kontaktnahme gewährleistet sein muss, können auch keine Porositätsgrade erzielt werden, die wesentlich oberhalb von 50 vol-% liegen. Damit scheidet eine solche Herstellungsweise a priori für viele funktionelle Anwendungen, z.B. auf dem Gebiet der medizinischen Implantate aus.

In der keramischen Sintertechnik, beispielsweise bei der Erzeugung von Schleifscheiben, werden deshalb oft Partikel einer zweiten Phase zusammen mit einem Binder zugemischt, die bei tieferen Temperaturen aus dem Gemisch entfernt werden und dabei ein Netzwerk von Hohlräumen zurücklassen. Zu diesem Zweck werden den abrasiven Partikeln vor dem Sintern beispielsweise Naphtalin-Kugeln zugemischt. Diese Kugeln geben ein Hohlvolumen vor, um welches sich die feineren Partikeln der keramischen Phase anordnen. Da die Naphtalin-Kugeln schon bei Temperaturen unterhalb von 100°C verdampfen, haben die Kugeln also nur die Funktion der Bereitstellung eines Porenvolumens im Grünkörper, der nachfolgende Sintervorgang muss so geführt werden, dass die Dimensionstreue für die Trennscheibe in gewünschten Grenzen eingehalten wird, was ein grosses Mass an empirischer Erfahrung bei der Wahl von Zeit und Temperatur verlangt.

Werden beim Aufbau eines porösen Formkörpers aus Metall Binder zur Erzeugung eines Grünkörpers eingesetzt, so müssen diese schon bei sehr tiefen Temperaturen, bei denen noch keine Gefahr der Aufnahme von Binderbestandteilen durch die metallische Matrix besteht, unter reduzierenden und/oder neutralen atmosphärischen Bedingungen entfernt werden können, denn im Gegensatz zu keramischen Systemen ist ein oxidatives Ausbrennen in der Regel hier nicht zulässig. Solche Bindersysteme werden meist aus der Gruppe der organischen Verbindungen gewählt und sie können bei Temperaturen unterhalb von 200 - 400°C durch Zersetzen und/oder Verdampfen aus dem Sinterkörper praktisch spurlos aus dem Sinterkörper entfernt werden. Dazu gehören thermoplastische Binder, wie Paraffine oder Wachse, teilweise in Mischung mit Polymeren wie z.B. PE, PP und weiteren Additiven wie z.B. Karbonsäuren und ihren Estern, duroplastische Binder, z.B. Furanharze oder auch Gelbinder, wie z.B. wässrige Lösungen von Methylzellulose.

Beim üblichen Sintervorgang geht es vor allem darum, zu einem möglichst dichten metallischen Körper zu kommen. Aus diesem Grund spielt es keine Rolle, dass die Binderphase den Formkörper schon bei sehr tiefen Temperaturen verlässt, ihr Verschwinden ist geradezu die Voraussetzung für die Erlangung einer möglichst hohen Dichte. Für den normalen Prozess genügt völlig, dass der binderlose Grünkörper gerade noch eine rudimentäre Festigkeit besitzt, um seine Form beizubehalten. Soll jedoch eine offenporige und vor allem dünnwandigen Struktur erzielt werden, so kehrt sich das Verschwinden des Binders und der porenbildenden Phase mit ihrer die Struktur stützenden Wirkung ins Gegenteil um. Die Porenstruktur ist nun schutzlos der Wirkung der Oberflächenspannung ausgesetzt und der Formkörper beginnt zu schwinden, wobei die Zellwände immer dicker werden und Durchgangskanäle in zunehmendem Masse verschlossen werden. Die endgültige Geometrie des Formkörpers ist hier eine Funktion der lokalen Porengeometrie (Krümmungsradius) sowie von Zeit und Temperatur und ist daher nicht exakt beherrschbar.

Nach US 5'034'186 SHIMAMUNE ET ALII existiert ein Verfahren, bei dem eine hochporöse Deckschicht auf einer Titanelektrode hergestellt wird, indem ein feines Titanpulver mit bis zu 75 vol-% Magnesiumpulver gemischt wird. Im Gegensatz zu thermisch instabilen organischen Porenbildnern verbleiben die Magnesiumpartikel zwar bei höheren Temperaturen im Gemisch und üben dadurch eine stabilisierende Wirkung aus, jedoch kann der dabei entstehende Magnesiumdampf infolge seiner hohen Reaktivität z.B. mit sauerstoff- oder stickstoffhaltigen Zersetzungsprodukten, welche Magnesiumoxid oder -nitrid zu einem starken Schwellen der Porenhohträume führt, was Anlass zur Bildung von Mikrorissen sein kann.

Aus der DE-A 2 256 716 GOETZEWERKE ist ein Verfahren zur Herstellung von porösen Sinterwerkstoffen bekannt, bei welchem der Hauptbestandteil des Sinterpulvergemisches aus Eisen oder einer Eisenlegierung besteht und dem ein lösliches Salz beigegeben wird, welches nach erfolgter Sinterung herausgelöst werden kann. Die Verwendung von Eisen oder Eisenlegierungen als hauptsächlicher Metalllieferant hat allerdings den Nachteil, dass Körner aus solchen Stoffen rasch oxidieren und mit einem mehr oder weniger dicken keramogenen Film bedeckt sind. Dies wiederum bewirkt, dass die Oberflächendiffusionsprozesse, welche den Sinterprozess an der Kontaktstelle der einzelnen Körner einleiten, diese Trennschichten überwinden müssen, was zu einer nachteiligen Erhöhung der Sintertemperatur führt.

Die Erfindung will Abhilfe für alle oben aufgezählten Nachteile schaffen. Die Erfindung löst das Problem gemäss den Merkmalen des Anspruchs 1.

Durch Verwendung von Hydrid-Partikeln wird die Sintertemperatur erniedrigt, so dass eine bessere Beibehaltung der inneren Oberfläche und Volumina erzielt wird. Die Hyride zerfallen verhältnismässig leicht und ihr Zerfallsprodukt ist Wasserstoff, das ein reduzierendes und rasch entfernbares Gas ist. Somit wird die Herstellung von offenporigen metallischen Strukturen effizienter.

Besonders ist dabei, dass die erste Phase Hydride zumindest eines der Metalle umfasst, welche die gesinterte Legierung bilden, da sie einerseits thermisch instabil sind und dadurch verhältnismässig leicht zerfallen und andererseits ihr Zerfallsprodukt das reduzierende Gas Wasserstoff ist, welcher infolge seiner hohen Diffusionsgeschwindigkeit bei ausreichend tiefem Wasserstoffpotential der Umgebung, etwa durch Einwirkung von Vakuum oder Inertgas, rasch aus der sinternden Metallphase entfernbar ist.

Zu den interstitiell in einer Metallphase löslichen Elementen gehören Kohlenstoff, Sauerstoff, Stickstoff, Schwefel und Phosphor. Es ist jedoch zulässig für das Sintern von Titanstrukturen z.B. Calciumoxid als zweite Phase zu verwenden, da Calciumoxid den Sauerstoff so fest in Bindung hat, dass Titan nicht in der Lage ist, der zweiten Phase diesen Sauerstoff zu entziehen, was zur Versprödung führen würde.

Gemäss einer bevorzugten Ausführungsform der Erfindung werden Kochsalz, Calciumfluorid sowie Kryolithe zum Aufbau der zweiten Phase verwendet.

Die Teilchen der ersten Phase bestehen aus Metallverbindungen. Bei einer speziellen Ausführungsform können auch thermisch instabile Oxide oder Nitride gewählt werden, welche unter Sinterbedingungen zum Metall zerfallen.

Bei einer bevorzugten Ausführungsform der Erfindung besteht die erste Phase aus Titanhydrid. Als Material für die zweite Phase werden erfindungsgemäss Alkali- oder Erdalkalihalogenide verwendet. Diese Verbindungen enthalten weder Kohlenstoff, Stickstoff oder Sauerstoff, welche ja bekannterweise von Titan unter starker Abnahme der Zähigkeit gierig aufgenommen werden. Diese Halogenide sind ja teilweise auch aus dem normalen Herstellungsgang von Titan bekannt. So tritt z.B. Magnesiumchlorid als Reaktionsprodukt der Reaktion von Titanchlorid mit Magnesium neben dem Hauptprodukt Titanschwamm im Kroll-Prozess als inertes Nebenprodukt auf oder bei einem anderen Reduktionsverfahren entsteht Natriumjodid gemeinsam mit Titan als Produkt der Zersetzung von Titanjodid.

Bezüglich der Geometrie und dem Aufbau der beiden Phasen, welche im Gemisch den Formkörper bilden, können diese direkt aus Partikeln der beiden Phasen bestehen, welche miteinander vermischt werden (in diesem Falle ist der Ausdruck "Teilchen" mit den entsprechenden Partikeln, welche diese Phase aufbauen, identisch). Es besteht jedoch auch die Möglichkeit, die Partikeln einer Phase vorgängig der Vermischung mit der anderen Phase zuerst mit einem geeigneten Binder zu einfachen Agglomeraten, z.B. Kugeln oder auch zu komplexeren Geometrien zu formen und erst dann mit diesen Teilchen ein Gemisch mit den Teilchen der anderen Phase aufzubauen, deren Partikel vorgängig ebenfalls wahlweise z.B. agglomeriert wurden. Wird ein Binder für den Aufbau der Teilchen einer Phase verwendet, welcher beim Sintervorgang schädliche Zersetzungsprodukte an die Metallphase abgeben würde, muss er - genau wie dies für die Bindersysteme gilt, welche zur Verbindung der beiden übergeordneten Phasen gilt, schon bei Temperaturen unterhalb des Sintervorgangs aus dem Gemisch entfernbar sein. In der Regel werden deshalb auch für diesen Zweck organische Binderphasen zur Anwendung kommen, wie sie weiter oben in Analogie zum Spritzgiessprozess beschrieben wurden.

Wie oben gesagt, besteht das Hauptziel des erfindungsgemässen Verfahrens darin, eine feste metallische Zellstruktur unter Beibehaltung der inneren Oberflächen und Volumina zu erzielen. Dieses Ziel wird vor allem dadurch erreicht, indem die zweite, das Leervolumen bildende Phase auch bei der Sintertemperatur noch eine mechanische Stützwirkung ausübt. Generell kann gesagt werden, dass die Effizienz dieses Vorgangs sich verbessert, bei je tieferen Temperaturen der Sintervorgang zu einer dichten Zellstruktur führt. Dies kann entsprechend dem ersten Anspruch dadurch geschehen, indem neben Metallpulvern auch leicht zersetzliche Metallverbindungen für die erste Phase eingesetzt werden. In der Sintertechnik von Titanpulvem wird Titanhydrid manchmal als Sinterhilfsmittel eingesetzt. Während normale Titanpulver zur dichten Versinterung meist Temperaturen im Bereich von 1200 - 1300°C benötigen, zerfallen feinkörnige Titanhydrid-Pulver je nach Wasserstoffpartialdruck der Umgebung bereits bei Temperaturen oberhalb von 600°C und das in statu nascendi frisch gebildete Titan kann schon bei Temperaturen unterhalb von 800°C ansintem.

Dieser frühzeitige Vorgang des Ansintems kann noch weiter unterstützt werden, wenn man bei geeigneten Legierungen die Technologie des Flüssigphasesintems einsetzt. Gemäss einer bevorzugten Ausführungsform der Erfindung kann zumindestens ein Teil der Partikel der ersten Phase, d.h. Metalle, Metallegierungen oder Metallverbindungen, unter Verwendung eines der bekannten Beschichtungsverfahren, mit einem tiefschmelzenden Legierungselement beschichtet werden, wobei dieses Element nach dem Sintervorgang in der für die angestrebte Legierung korrekten Konzentration vorliegt. Besonders elegant kann dieses Prinzip durch Aluminium-Beschichtung von Titan- oder Titanhydrid-Partikeln, welche bereits mit Vanadin vorlegiert wurden, zur Erzeugung einer Struktur aus der bekannten Legierung Ti6Al4V eingesetzt werden. Bei Erreichung der Temperatur von 660°C kommt es zur Bildung einer flüssigen Aluminiumphase, welche sich an den Kontaktstellen der metallischen Partikel infolge der Grenzflächenspannung zusammenzieht. Bei weiterer Erhitzung erfolgt eine zumnehmende Zulegierung des Titans und damit kommt es schon bald zu einer festen Bindung zwischen den Partikeln der ersten Phase.

Es ist zweckmässig nur einen Teil der Partikel zu beschichten. Da bei kleineren Partikeln schon geringe Beschichtungsdicken zu einer eventuell zu grossen Konzentrationszunahme am betreffenden Element führen würden, können z.B. nur 10% der Partikel beschichtet werden, womit ein Verdünnungseffekt erzielt wird. Z.B. würde der Versuch, eine Titanlegierung mit 6% Aluminium herzustellen, zu äusserst dünnen Aluminiumschichten führen.

Gemäss einer bevorzugten Ausführungsform der Erfindung kann die Erzeugung von formkörpem auch binderlos nach dem Schüttsinterverfahren erfolgen, indem die gut vermischten Teilchen der ersten und zweiten Phase eventuell unter Zuhilfenahme verdichtender Massnahmen, z.B. einem Vibrator, in eine bei der Sintertemperatur thermisch und chemisch stabile Form eingebracht wenden. Handelt es sich dabei um eine chemisch stark reaktive Metallphase, z.B. Titan oder seine Legierungen, welche bekanntlich die meisten keramischen formen angreifen, dann kann eine solche form auch aus einem der Materialien bestehen, wie sie entsprechend Anspruch 4 für die zweite Phase beansprucht werden, also z.b. Kochsalz. Eine solche Kochsalzform ist bis zu einer Temperatur von 801° beständig. Erst oberhalb dieser Temperatur schmilzt diese Form, genauso wie die im Inneren des Formkörpers befindlichen Salzteilchen, wobei der solcherart innen und aussen freigestellte Formkörper nach ausreichender Vorsinterzeit bei dieser Temperatur schon bereits eine ausreichende Eigenfestigkeit erreicht hat. Selbstverständlich können für diesen Schüttsintervorgang bei weniger reaktiven Metallen jedoch auch Dauerformen aus Keramik oder Metall eingesetzt werden. Im Falle einer metallischen Dauerform muss deren Oberfläche natürlich - zur Verhinderung einer Diffusionsverschweissung - entsprechend geschlichtet werden.

Gemäss einer bevorzugten Ausführungsform der Erfindung kann der aus der ersten und zweiten Phase bestehende Formkörper auch durch eine dritte Binderphase zu einem Grünkörper stabilisiert werden, welche in Analogie zum Pulverspritzgiessen vor dem eigentlichen Sintervorgang entweder durch Zersetzung und/oder Verdampfung unter Hinterlassung einer ausreichenden Grünfestigkeit aus dem Formkörper entfernt wird. Im Gegensatz zum Puiverspritzgiessen, wo diese Binderphase den Raum zwischen den Metallpartikeln zur Erzielung günstiger rheologischer Eigenschaften weitgehend ausfüllt, was zu einem langwierigen und schwierigen Entwachsungsvorgang führt, kann die Binderphase beim erfindungsgemässen Verfahren meist in viel geringerer Konzentration verwendet werden, denn sie muss nur dafür sorgen, dass die Partikel der beiden Phasen an den Kontaktstellen miteinander verklebt sind, sodass ein ausreichend fester Formkörper entsteht. Beim Entgasungsvorgang des Binders sind deshalb ausreichend dimensionierte Kanäle vorhanden, sodass dieser Vorgang einfach und schnell vonstatten gehen kann. Seine Dauer ist lediglich an die Mindestzeit gebunden, welche zur Erlangung einer ausreichenden Grünfestigkeit benötigt wird. Wird allerdings das Spritzgussverfahren zur Erzeugung von Formkörpem eingesetzt, dann gelten für den Binder mengenmässig die üblichen Bedingungen, die für die Erzielung einer ausreichenden Spritzfähigkeit in der Spritzgussmaschine nötig sind. Dies führt dann natürlich auch zu einem entsprechend verlangsamten Entwachsungsvorgang.

Das erfindungsgemässe Verfahren soll im folgenden anhand mehrerer Ausführungsbeispiele beschrieben werden.

### Beispiel 1 (gehört nicht zur Erfindung)

In einem ersten Beispiel wird die Herstellung einer offenporigen Metallstruktur in Form eines Zylinders mit 20 mm Durchmesser und 80 mm Länge aus einem rostfreien Stahl beschrieben. Zu diesem Zweck wurde ein Stahlpulver mit der Zusammensetzung von etwa 18 m-% Chrom und 9 m-% Nickel und einer mittleren Korngrösse von 20 µm mit 80 vol-% annähernd kugelförmigen Calciumcarbonat-Teilchen mit einem mittleren Durchmesser von 0.2 mm unter Beigabe von 2 m-% eines organischen Paraffin in einer Matritze zu Formkörpern gepresst. Die Formkörper wurden anschliessend in einen Schutzgasofen eingesetzt und dem folgenden Temperaturprogramm unterworfen:

| | | |
|---|---|---|
| 3h | 25 - 300°C | strömendes Argon |
| 3h | 300 - 1100°C | strömendes Formiergas (93% Argon, 7% Wasserstoff) |
| 6h | 1100°C | strömendes Formiergas |

Im Temperaturbereich von 900°C kam es dabei zur Zersetzung des Calciumcarbonats zu gebranntem Kalk unter Abgabe von Kohlensäure. Dieser Prozess war mit einer nur leichten Schwindung der Calciumoxid-Partikeln verbunden, der wesentliche Volumsverlust entstand durch die Bildung feinster Porenkanäle in den Calciumoxid-Partikeln. Der Durchmesser und die Länge des Zylinders nahmen deshalb nur sehr wenig gegenüber dem Ausgangszustand ab. Nach Abschluss des Sinterprozesses wurde die gesinterte Probe abgekühlt und dem Ofen entnommen. Der Formkörper wurde anschliessend in stark verdünnte und lebhaft gerührte Salzsäure während mehrerer Tage eingelegt, wonach das Calciumoxid völlig hydratisiert und aus dem Formkörper verschwunden war. Als Resultat entstand ein hochfester und -zäher Formkörper mit einer relativen Dichte von 32 % bezogen auf die Dichte von rostfreiem Stahl.

### Beispiel 2 (gehört nicht zur Erfindung)

In einem zweiten Beispiel wird die Herstellung einer offenporigen Struktur in Form eines rostfreien Stahlfilters mit 8 mm Höhe und einem G1/8" Aussengewinde beschrieben, wobei die Porengrösse jedoch hier fast um eine Zehnerpotenz kleiner ist.. Da im Filter gleichmässige und rundlich nach aussen gewölbte Hohlräume mit Porendurchmessem von maximal 35 µm gefordert werden, wäre der Einsatz von Metallpartikeln im Korngrössenbereich unterhalb von 500 nm nötig, denn nur so feine Pulver sind in der Lage, die feinen Zellwände rings um die volumsbildende Phase aufzubauen, welche im vorliegenden Falle ebenfalls aus Calciumcarbonat-Partikeln mit einem mittleren Durchmesser von 30 µm bestand. Solche rostfreien Stahlpulver sind jedoch auf dem Markt nicht erhältlich, ihre Herstellung wäre auch extrem teuer. Aus diesem Grunde wurde ein stöchiometrisch entsprechend der Legierung eingestelltes Gemisch von Eisen-, Chrom- und Nickeloxid verwendet, wobei die mittlere Korngrösse aller Partikel unterhalb von 500 nm lag. Dieses Pulver wurde als erste Phase mit den Calciumcarbonat-Kugeln homogen vermischt und anschliessend ohne Binder in eine dünnwandige Tonerdeform eingefüllt. Diese Form war vorgängig nach dem Lost-Wax-Verfahren durch Ausschmelzen eines entsprechenden Wachsmodells hergestellt worden worden. Diese Form wurde in einen Schutzgasofen eingesetzt und anschliessend wurde das folgende Temperatur-Zeit-Programm gefahren:

| | | |
|---|---|---|
| 5h | 25 - 1350°C | strömender pulsierender technischer Wasserstoff |
| 3h | 1350°C | strömender pulsierender Reinstwasserstoff |

Um zu gewährleisten, dass der Reduktionsprozess der Oxide mit guter Kinetik ablief, wurde der Druck des Wasserstoff, der normalerweise bei 1050 mbar lag, alle 2 Minuten während 10 Sekunden auf 1500 mbar angehoben. Durch diese Massnahme wird der bei der Reduktion entstehende Wasserdampf verdünnt und es gelangt immer wieder frischer Wasserstoff an die inneren Oberflächen des Formkörpers. Nach der Abkühlung wurde der Formkörper dem Ofen entnommen und wiederum in verdünnter Säure ausgewaschen. Infolge der Tatsache, dass es bei der Reduktion von Metalloxiden zu einer starken Volumsverminderung kommt, die Calciumoxid-Partikel jedoch weitgehend ihre äussere Form behielten, kam es nur zu einem geringen Schwund des Formteils, jedoch zu einer Zunahme des Porositätsgrades, da die Verbindungsstege der metallischen Struktur infolge des starken Schwundes bei der Reduktion einen dünneren Querschnitt hatten. Das Schliffbild des metallischen Gefüges zeigte eine von nichtmetallischen Einschlüssen weitgehend freie austenitische Matrix, was sich auch durch die guten Zähigkeitseigenschaften des Formkörpers bestätigte.

### Beispiel 3

In einem dritten Beispiel wird die Herstellung eines medizinischen Implantats für einen Hüftgelenkprothesenkopf aus Reintitan beschrieben. Zu diesem Zweck wurde ein Gemisch von 60.7 m-% Kochsalzkugeln, deren Durchmesser zwischen 0.6 und 0.85 mm ausgesiebt worden war, mit 21.3 m-% Titanhydridpartikeln, die eine Komgrösse von 1 - 3 µm aufwies, zusammen mit 18% einer gesättigten wässrigen Kochsalzlösung mit 5% Isopropylalkohol zu einer homogenen Paste angerührt. Diese Paste wurde in eine mit Metall hinterstützte zweiteilige Silikongummiform unter Vibration eingefüllt und anschliessend in einem Trockenschrank bei 80°C während 24 h getrocknet. Das entformte Teil wurde anschliessend in einen Schutzgasglühofen eingebracht, wobei anschliessend das folgende Zeit-Temperatur-Programm zur Anwendung kam:

| | | |
|---|---|---|
| 6h | 25 - 300°C | Pulsierendes Argon (1000/1500 mbar) |
| 2h | 300 - 780°C | Pulsierendes Argon (30/10 mbar) |
| 10h | 780°C | Pulsierendes Argon (30/10 mbar) |
| 2h | 780 - 1100°C | Pulsierendes Argon (30/10 mbar) |
| 5h | 1100°C | Pulsierendes Argon (30/10 mbar) |

Während der ersten Aufheizung bis 300°C wird das Wasser zusammen mit dem organischen Bindemittel entfernt. Dabei zeigt sich, dass Titanhydrid sich gegenüber Wasser und dem Binder völlig inert verhält. Dies deckt sich mit Erkenntnissen der Literatur, wo beschrieben wird, dass Titanhydrid - solange es bei seiner Zersetzung Wasserstoff in grösseren Mengen abgibt - gegen das Eindringen fremder Gase völlig geschützt ist. Die Zersetzung des Titanhydrids beginnt unter den obigen Versuchsbedingungen knapp unterhalb von 600°C und ist bei 780°C weitgehend abgeschlossen. Gleichzeitig setzt der Sinterprozess an den Kontaktstellen der frisch gebildeten Titanpartikel ein. Die Temperatur von 780°C für das Konstanthalten der Temperatur wurde gewählt, da diese noch knapp unterhalb der Schmelztemperatur des Kochsalzes von 801°C liegt, sodass die Struktur während 10 h ohne Schwund versintem kann. Anschliessend wurde während 2 Stunden auf 1100°C erhitzt, wobei das Salz schmolz und weitgehend aus dem Formkörper ausfloss. Während der weiteren 5h verdampfte noch der grösste Teil des Restes des Kochsalzes, wobei es gleichzeitig zu einem linearen Schwund von 18% kam, der sich jedoch gleichmässig in alle Richtungen auswirkte. Der fast vom Kochsalz befreite Formkörper wurde anschliessend in fliessendem Wasser während mehrerer Stunden gespült. Als Resultat entstand eine Struktur mit 79.5 vol-% Porosität, und annähernd im Sinne einer dichtesten Kugelpackung regelmässig verteilten offenen Poren mit einer mittleren Grösse von 0.62 mm. Mechanische Versuche zeigten, dass diese Struktur ein sprödes Verhalten zeigte, was darauf hindeutete, dass das Titan einen zu hohen Sauerstoffgehalt in Lösung hatte. Dies ergibt sich aufgrund der hohen Reaktivität des Titans, d.h. schon geringe Verunreinigungen des Titanhydrids, welches an der Oberfläche dünne Oxidschichten enthält, genügen für diese Verminderung der mechanischen Eigenschaften, da ja ein solcher Formkörper eine riesige innere Oberfläche aufweist. Entsprechend dem Anspruch 17 wurde der Formkörper zusammen mit Calciumgranulat in einer vakuumevakuierten dünnwandigen Stahlretorte in den Schutzgasofen eingebracht und während 4 h auf 1000°C gebracht. Der dabei entwickelte Calciumdampfdruck erzeugte ein tiefes Oxidationspotentials an den inneren Oberflächen des Formkörpers, sodass der Sauerstoff aus den dünnen Zellwänden über den Vorgang der Festkörperdiffusion nach aussen diffundierte, wo er mit dem Dampf zu Calciumoxid reagierte. Im Anschluss an diese Behandlung wurde der Formkörper wiederum mit verdünnter Salzsäure gewaschen und dieser Titankörper zeigte ein ausgezeichnetes Zähigkeitsverhalten.

### Beispiel 4

Ein viertes Beispiel zeigt einen Fall, bei dem eine Komponente aus Voiltitan mit einer Deckschicht aus porösem Titan beschichtet wurde. Zu diesem Zweck wurden feine Partikel aus Calciumfluorid mit einem Alginat zu Kugeln mit einem Durchmesser von 0.5 mm agglomeriert. Diese Kugeln wurden anschliessend mit einem Gemisch von 90 m-% Reintitanpulver, welches eine mittlere Komgrösse von 15 µm hatte und 10 m-% Titanhydrid unter Zugabe eines Alkohol/Wachsgemisches zu einer dickflüssigen Paste angeteigt. Diese Paste wurde anschliessend manuell in einer etwa 3 mm dicken Schicht auf die Elektrode aufgebracht und anschliessend während 48 h im Trockenschrank bei 110°C getrocknet. Anschliessend wurde das folgende Temperatur-Zeit-Programm gefahren:

| | | |
|---|---|---|
| 6h | 25 - 300°C | Pulsierendes Argon (1000/1500 mbar) |
| 2h | 300 - 1250°C | Pulsierendes Argon (30/10 mbar) |
| 2h | 1250°C | Pulsierendes Argon (30/10 mbar) |

Da Calciumfluorid erst bei Temperaturen oberhalb von 1400°C schmilzt, verblieben die Partikel im Inneren des Formkörpers und mussten deshalb nach dem Sintervorgang durch chemische Lösung entfernt werden. Hier zeigte sich jedoch der Vorteil, dass die Calciumfluorid-Teilchen vorgängig aus viel feineren Partikeln zu kugelförmigen Körpern agglomeriert worden waren. Nachdem das Alginat sich bei Temperaturen unterhalb von 300°C zersetzt hatte, wurden die dadurch entstehenden lockeren Haufwerke aus Calciumfluorid-Partikeln durch die sie umgebenden Titan- und Titanhydrid-Partikel am Ort gehalten. Sobald jedoch der Sintervorgang der Titanphase einsetzt, der in diesem Beispiel bei wesentlich höherer Temperatur viel schneller ablief, gelangten die Calcium-Partikel-Haufwerke unter kompressiven Druck und verhinderten dadurch, dass die Oberflächenspannung zu einer starken Schwindung der Struktur führte. Anschliessend an den Sintervorgang wurde der abgekühlte Titankörper während mehrerer Tage in einer gut gerührten Komplexon-Lösung gespült, wobei es zur langsamen Auflösung der Calciumfluorid-Partikel kam.

## Patentansprüche

1. Gemisch aus zwei Teilchen-Phasen zur Herstellung eines bei höheren Temperaturen sinterfähigen Grünlings, wobei
A) die erste Phase Teilchen enthält, welche aus einer oder mehreren Metallverbindungen bestehen;
B) die zweite Phase Teilchen aus der Gruppe der anorganischen Verbindungen enthält, welche oberhalb von 400°C keine Zersetzungsprodukte freisetzen, die in der sinternden Metallphase interstitiell löslich sind und/oder mit ihr zu stabilen Verbindungen reagieren; und
C) die anorganischen Verbindungen aus der Gruppe der Alkali- oder ErdalkaliHalogenide ausgewählt ist,
**dadurch gekennzeichnet, dass**
D) die erste Phase thermisch instabile Hydride zumindest eines der Metalle umfasst, welche die gesinterte Legierung bilden.

2. Gemisch nach Anspruch 1, **dadurch gekennzeichnet, dass** die erste Phase Partikel aus Titan umfasst.

3. Gemisch nach Anspruch 1 oder 2, **dadurch gekennzeichnet, dass** die erste Phase des Gemisches zusätzlich noch Teilchen enthält, welche aus einem Metall bestehen.

4. Gemisch nach Anspruch 1 oder 2, **dadurch gekennzeichnet, dass** die erste Phase des Gemisches zusätzlich noch Teilchen enthält, welche aus einer Metallegierung bestehen.

5. Gemisch nach einem der Ansprüche 1 bis 4, **dadurch gekennzeichnet, dass** die anorganischen Verbindungen aus folgender Gruppe ausgewählt werden: NaCl, CaF₂, K₃AlF₆ und Na₃AlF₆.

6. Gemisch nach einem der Ansprüche 1 bis 5, **dadurch gekennzeichnet, dass** die Körper der ersten und/oder der zweiten Phase Agglomerate oder Formkörperchen von Partikeln sind welche durch einen Binder in Form gehalten werden, der bei Temperaturen unterhalb des Sinterprozesses zersetzt und/oder verdampft.

7. Gemisch nach einem der Ansprüche 1 bis 6, **dadurch gekennzeichnet, dass** die erste Phase thermisch instabile Oxide zumindest eines der Metalle umfasst, welche die gesinterte Legierung bilden.

8. Gemisch nach einem der Ansprüche 1 bis 6, **dadurch gekennzeichnet, dass** die erste Phase thermisch instabile Nitride zumindest eines der Metalle umfasst, welche die gesinterte Legierung bilden.

9. Gemisch nach einem der Ansprüche 1 bis 8, **dadurch gekennzeichnet, dass** die erste Phase Titanhydrid-Partikel umfasst.

10. Gemisch nach einem der Ansprüche 1 bis 9, **dadurch gekennzeichnet, dass** zumindest ein Teil der Partikel der ersten Phase mit einem Überzug aus einem Metall versehen wird, welcher im Kontakt mit anderen Bestandteilen der ersten Phase zumindest zu Beginn des Sintervorgangs eine niedrigschmelzende Legierung bildet und dass die Konzentration dieses Metalls nach Abschluss des Sintervorgangs dem gewünschten Wert in der Legierung entspricht.

11. Gemisch nach einem der Ansprüche 1 bis 10, **dadurch gekennzeichnet, dass** es zusätzlich zur ersten und zweiten Phase eine dritte Phase in Form eines organischen oder anorganischen Binders in einer Zusammensetzung enthält, wie sie beim Pulverspritzguss zur Anwendung kommt.

12. Verfahren zur Herstellung eines bei höheren Temperaturen sinterfähigen Grünlings unter Verwendung des Gemisches nach einem der Ansprüche 1 - 11, **dadurch gekennzeichnet, dass** die erste und zweite Phase des Gemisches homogen vermischt werden und danach dieses Gemisch in eine bei Sintertemperatur thermisch und chemisch stabile Form eingebracht wird.

13. Verfahren zur Herstellung von metallischen Formkörpern mit offener Porosität, wobei ein bei höheren Temperaturen sinterfähiger Grünling durch das Verfahren nach Anspruch 12 hergestellt wird, **gekennzeichnet durch** folgenden Verfahrensschritt: Erwärmung des Grünlings bis zum Versintern der Partikel der ersten Phase zu einer offenporigen Struktur, wobei die Partikel der zweiten Phase während dem oder im Anschluss an den Sintervorgang aus den Poren des Formkörpers entfernt werden.

14. Verfahren nach Anspruch 13, **dadurch gekennzeichnet, dass** die Entfernung der Partikel der zweiten Phase oberhalb von 400°C vor oder während dem Sinterprozess erfolgt.

15. Verfahren nach Anspruch 13, **dadurch gekennzeichnet, dass** die Entfernung der Partikel der zweiten Phase nach dem Sintervorgang durch Herauslösen mit einem Lösungsmittel erfolgt.

16. Verfahren nach einem der Ansprüche 13 - 15, **dadurch gekennzeichnet, dass** der versinterte Formkörper mit einem flüssigen und/oder dampfförmigen Alkali- oder Erdalkalimetall behandelt wird.

## Claims

1. A mixture of two particulate phases to be used in the production of a green compact that can be sintered at higher temperatures, whereby
A) the first phase contains particles that consist of a metal compound;
B) the second phase contains particles selected from the group of the inorganic compounds which at temperatures beyond 400 DEG C do not release any decomposition products that are interstitially soluble in the sintering metal phase and/or react with said phase to form stable compounds; and
C) that the inorganic compounds are selected from the group of the alkali halogenides or alkaline earth halogenides,
**characterised in that**
D) the first phase comprises thermally unstable hydrides of at least one of the metals which form the sintered alloy.

2. A mixture as claimed in claim 1, **characterised in that** the metal compound is a titan compound.

3. A mixture as claimed in claim 1 or 2, **characterised in that** the first phase of the mixture additionally comprises particles which consist of a metal.

4. A mixture as claimed in claim 1 or 2, **characterised in that** the first phase of the mixture additionally comprises particles which consist of a metal alloy.

5. A mixture as claimed in any one of the claims 1 to 4, **characterised in that** the inorganic compounds are selected from the following group: NaCl, CaF₂, K₃AlF₆ and Na₃AlF₆.

6. A mixture as claimed in any of the claims 1 to 5, **characterised in that** the bodies of the first and/or the second phase are agglomerates or shaped corpuscles of powder particles which are kept in place by means of a binder that disintegrates and/or evaporates at temperatures below the beginning of the sintering process.

7. A mixture as claimed in any of the claims 1 to 6, **characterised in that** the first phase comprises oxides of at least one of the metals which form the sintered alloy.

8. A mixture as claimed in any of the claims 1 to 6, **characterised in that** the first phase comprises nitrides of at least one of the metals which form the sintered alloy.

9. A mixture as claimed in any of the claims 1 to 8, **characterised in that** the first phase comprises particles consisting of titanium hydride.

10. A mixture as claimed in any of the claims 1 to 9, **characterised in that** at least part of the particles of the first phase are provided with a metal coating which in contact with the other components of the first phase form, at least at the beginning of the sintering process, a low melting point alloy and that after termination of the sintering process the concentration of this metal in the alloy corresponds to the desired value.

11. A mixture as claimed in any of the claims 1 to 10, **characterised in that** in addition to the first and second phases it contains a third phase in the form of an organic or inorganic binder in a composition corresponding to that used in powder injection moulding.

12. A method for producing a shaped body that can be sintered at higher temperatures as claimed in any of the claims 1 to 11, **characterised in that** the first and second phases composing the mixture are homogeneously mixed and that subsequently said mixture is inserted into a mould which at the sintering temperature is thermally and chemically stable.

13. A method for producing shaped metal bodies with interconnecting pore structures by using the shaped body capable of being sintered as claimed in claim 14, **characterised by** the following procedure step: heating of the green compact until the particles of the first phase are sintered so as to form an interconnecting pore structure, the particles of the second phase being eliminated from the pores of the shaped body during or subsequent to the sintering process.

14. A method as claimed in claim 13, **characterised in that** the elimination of the particles of the second phase takes place prior to or during the sintering process at a temperature beyond 400 DEG C.

15. A method as claimed in claim 13, **characterised in that** the elimination of the particles of the second phase takes place subsequent to the sintering process by dissolving out said particles using a solvent.

16. A method as claimed in any of the claims 13 to 15, **characterised in that** after having undergone the sintering process, the shaped body is treated with a liquid and/or a vaporous alkali metal or alkaline earth metal.

## Revendications

1. Mélange de deux phases particulaires pour la production d'un corps vert apte au frittage à des températures élevées, dans lequel
A) la première phase contient des particules qui sont constituées d'un ou plusieurs composés métalliques,
B) la deuxième phase contient des particules du groupe des composés inorganiques qui ne libèrent pas au-dessus de 400 °C des produits de décomposition qui ont une solubilité interstitielle dans la phase métallique qui fritte et / ou qui réagissent avec elle pour former des liaisons stables, et
C) les composés inorganiques sont choisis dans le groupe des halogénures alcalins ou alcalino-terreux,
**caractérisé en ce que**
D) la première phase comprend des hydrures thermiquement instables d'un des métaux qui forment l'alliage fritté.

2. Mélange selon la revendication 1, **caractérisé en ce que** la première phase comprend des particules de titane.

3. Mélange selon la revendication 1 ou 2, **caractérisé en ce que** la première phase du mélange comprend encore en plus des particules qui sont constituées d'un métal.

4. Mélange selon la revendication 1 ou 2, **caractérisé en ce que** la première phase du mélange comprend encore en plus des particules qui sont constituées d'un alliage métallique.

5. Mélange selon une des revendications 1 à 4, **caractérisé en ce que** les composés inorganiques sont choisis dans le groupe suivant : NaCl, CaF₂, K₃AlF₆ et Na₃AlF₆.

6. Mélange selon une des revendications 1 à 5, **caractérisé en ce que** les corps de la première et / ou de la deuxième phase sont des agglomérats ou des petits corps façonnés de particules qui sont maintenus en forme par un liant qui se décompose / ou se volatilise à des températures en dessous du processus de frittage.

7. Mélange selon une des revendications 1 à 6, **caractérisé en ce que** la première phase comprend des oxydes thermiquement instables d'au moins un des métaux qui forment l'alliage fritté.

8. Mélange selon une des revendications 1 à 6, **caractérisé en ce que** la première phase comprend des nitrures thermiquement instables d'au moins un des métaux qui forme l'alliage fritté.

9. Mélange selon une des revendications 1 à 8, **caractérisé en ce que** la première phase comprend des particules d'hydrure de titane.

10. Mélange selon une des revendications 1 à 9, **caractérisé en ce qu'**au moins une partie des particules de la première phase est pourvue d'un revêtement dans un métal qui forme au contact avec d'autres constituants de la première phase au moins au début du processus de frittage un alliage à bas point de fusion et **en ce que** la concentration de ce métal correspond à la valeur souhaitée dans l'alliage au terme du processus de frittage.

11. Mélange selon une des revendications 1 à 10, **caractérisé en ce qu'**il contient, en plus de la première et de la deuxième phase, une troisième phase sous la forme d'un liant organique ou inorganique dans une composition comme celle qui est utilisée pour le moulage par injection de poudre.

12. Procédé pour la production d'un corps vert apte au frittage à des températures élevées en utilisant un mélange conforme à une des revendications 1 - 11, **caractérisé en ce que** la première et la deuxième phases du mélange sont mélangées de manière homogène et qu'ensuite, ce mélange est amené dans une forme thermiquement et chimiquement stable à température de frittage.

13. Procédé pour la production de corps façonnés en métal avec des pores ouverts, dans lequel un corps vert apte au frittage à des températures élevées est produit par le procédé selon la revendication 12, **caractérisé par** les opérations suivantes : chauffage du corps vert jusqu'au frittage des particules de la première phase pour former une structure à pores ouverts, les particules de la deuxième phase étant éliminées des pores du corps façonné pendant ou au terme du processus de frittage.

14. Procédé selon la revendication 13, **caractérisé en ce que** l'élimination des particules de la deuxième phase a lieu au-dessus de 400 °C avant ou pendant le processus de frittage.

15. Procédé selon la revendication 13, **caractérisé en ce que** l'élimination des particules de la deuxième phase a lieu par dissolution avec un solvant après le processus de frittage.

16. Procédé selon une des revendications 13 - 15, **caractérisé en ce que** le corps façonné fritté est traité avec un métal alcalin ou alcalino-terreux liquide et / ou à l'état de vapeur.
